(19) Europäisches Patentamt
European Patent Office
Office européen des brevets

(11) Publication number: **0 013 493 B1**

(12) **EUROPEAN PATENT SPECIFICATION**

(45) Date of publication of patent specification: **23.03.83**

(21) Application number: **79302866.3**

(22) Date of filing: **12.12.79**

(51) Int. Cl.$^3$: **C 07 C 119/042**, C 07 C 118/02, C 07 C 87/20, C 07 C 85/08, C 07 C 85/02, C 08 G 18/73, C 09 D 3/72

(54) **A novel aliphatic triisocyanate, a method for producing the same and a polyurethane coating composition prepared therefrom.**

(30) Priority: **29.12.78 JP 161804/78**

(43) Date of publication of application:
**23.07.80 Bulletin 80/15**

(45) Publication of the grant of the patent:
**23.03.83 Bulletin 83/12**

(84) Designated Contracting States:
**GB IT NL**

(56) References cited:
**DE - A - 2 012 755**
**FR - A - 2 306 979**

**CHEMICAL ABSTRACTS, vol. 72, no. 9, 2nd March 1970, page 358, no. 42743m Columbus, Ohio, U.S.A.**

**The file contains technical information submitted after the application was filed and not included in this specification**

(73) Proprietor: **TORAY INDUSTRIES, INC.**
**2, Nihonbashi-Muromachi 2-chome Chuo-ku**
**Tokyo 103 (JP)**

(72) Inventor: **Nishino, Masaki**
**480, 3-chome, Kizuki Nakahara-ku**
**Kawasaki-shi, Kanagawa-ken (JP)**
Inventor: **Yasuhara, Yutaka**
**82, 1-chome, Ikegamidai Midori-ku**
**Nagoya-shi, Aichi-ken (JP)**
Inventor: **Fukuda, Tadanori**
**1-2, Sakaemachi**
**Otsu-shi, Shiga-ken (JP)**
Inventor: **Sakamoto, Sadayuki**
**378, Ogawa, Shigaraki-cho**
**Koga-gun, Shiga-ken (JP)**

(74) Representative: **Ellis, John Clifford Holgate et al,**
**MEWBURN ELLIS & CO. 2/3 Cursitor Street**
**London EC4A 1BQ (GB)**

**EP 0 013 493 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

A novel aliphatic triisocyanate, a method for producing the same and a polyurethane coating composition prepared therefrom

This invention relates to a novel aliphatic triisocyanate, a method for producing the same and a polyurethane coating composition prepared therefrom.

It has long been known that aliphatic isocyanates impart premium properties to polyurethanes derived therefrom in terms of light stability. However, a conventional aliphatic isocyanate such as hexamethylene diisocyanate is characterized by toxicity which renders its handling and use extremely hazardous.

Accordingly, in order to lower the toxicity, they are often converted to polyisocyanates having a higher molecular weight before subjecting them to a practical use. Such a conversion may be carried out by reacting a relatively low molecular weight polyisocyanate with various polyfunctional compounds such as polyhydroxyl compounds and biuret derivatives. Examples of those converted polyisocyanates, as derived from hexamethylene diisocyanate, are as follows;

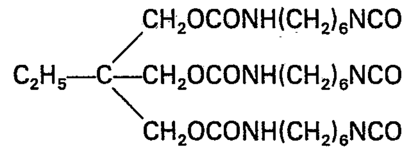

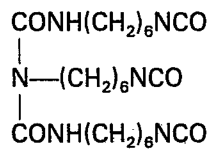

In such a conversion, however, a considerable amount of the starting isocyanates, hexamethylene diisocyanate in the above examples, tends to remain unreacted. As a result the mixtures thus obtained are often still toxic. Such conversion processes also involve a higher production cost. The converted polyisocyanates as mentioned above are usually so viscous that they require diluents for convenient handling and use, and may be unsuitable for making high solid type coatings.

Therefore, it has long been desired to produce polyisocyanates that have a low toxicity and viscosity, and that can give a non-yellowing polyurethane resin or coatings having excellent properties with respect to light stability, waterproofness, stability against organic and inorganic substances and so on.

We have now discovered a novel aliphatic triisocyanate, 1,6,11-undecanetriisocyanate represented by the following formula (I)

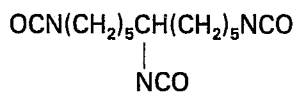 (I)

and have found that the triisocyanate has excellent properties as described below. The novel triisocyanate is produced by reacting 1,6,11-undecanetriamine, represented by the following Formula (II)

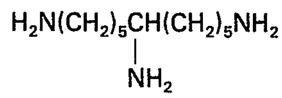 (II)

or its salts with phosgene. We also have found the novel triisocyanate can give an excellent novel polyurethane coating composition in combination with various polyols.

The starting triamine (II) can be produced by processes already known. For example, 7-(5'-aminopentyl)-3,4,5,6-tetrahydro-2H-azepine (hereinafter referred to as the "Schiff's base") represented by the following Formula,

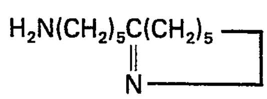

can be converted to the triamine by catalytic hydrogenation in the medium of an aqueous solution of ammonia.

The Schiff's base can be readily produced by thermolysis of ε-caprolactam, ε-aminocaptoic acid or an oligomer or polymer thereof in the presence of a hydroxide or oxide or an alkali metal or alkali earth metal such as lithium and calcium.

In place of the Schiff's base there can be used bis-(5-aminopentyl) ketone or a salt thereof, notably the hydrochloric acid salt.

The triamine thus produced is converted to the novel triisocyanate of the present invention by reacting with phosgene or other carbonyl dihalide. Phosgenation has previously been suggested (see GB 1 481 593) for producing an isomer mixture of alicyclic bicyclo triisocyanates, such as 2-isocyanatomethyl-3-(3-isocyanatopropyl)-5-isocyanatomethyl-bicyclo-[2,2,1]-heptane and its 6-isocyanatomethyl counterpart, though it has not, to the best of our belief, been employed to make an aliphatic triisocyanate.

In the method for making the triisocyanate of the invention, phosgene may be employed in either liquid or gaseous form. The amine may be supplied to the reaction zone in the form of amine salts formed with hydrogen chloride, sulfuric acid, phosphoric acid, carbonic acid or carboxylic acids such as acetic acid. The amine hydrochloride may be preferably employed. In a one-step method of manufacture the amine salt may be dispersed in an inert organic liquid reaction medium and phosgene added thereto preferably in stoichiometrical excess to the amino group existing in the reaction system, the temperature of the reaction medium being maintained within the range of from about 60°C to about 230°C, preferably about 180°C. In this one-step method, the amine hydrochloride or other salt should be in a pulverized form having an average size of below 1 mm, more preferably below 0.3 mm, since the amine salts scarcely dissolve in the reaction mixture.

Alternatively, the phosgenation of the amine can be carried out in a multi-step method. In a preferred embodiment of the invention, the phosgenation of the amine is first carried out in an inert organic liquid reaction medium at a temperature below 40°C, preferably below 8°C for a sufficient period to produce the corresponding carbamoyl chloride represented by the following Formula:

$$\begin{array}{l} ClOCNH(CH_2)_5CH(CH_2)_5NHCOCl \\ \qquad\qquad\qquad\quad | \\ \qquad\qquad\qquad NHCOCl \end{array}$$

Then the reaction mixture is heated, while being stirred, and maintained at a temperature in the range of from about 60°C to about 230°C, preferably in the range of about 100°C to about 180°C in the presence of phosgene. In the first step of said phosgenation, the reaction may be carried out by adding phosgene to the amine solution in an inert medium, or preferably by adding the solution of the amine in an inert medium to liquified phosgene or phosgene dissolved in an inert medium. Also in these cases, phosgene may be preferably used in a stoichiometrical excess to the amino groups. The carbamoyl chloride thus produced is not necessarily isolated, and may be subjected to the second step of the phosgenation in situ. This two-step procedure may have advantages over the one-step method because the one-step method tends to produce side products which result from the secondary reaction of the isocyanate with the starting amine.

In any procedure for phosgenation according to the invention, the molar ratio of phosgene to the amine hydrochloride group may be from about 1.1:1 to 10:1 and preferably at least 2:1. Suitable inert liquid reaction media for the phosgenation include aromatic hydrocarbons, chlorinated aromatic hydrocarbons and chlorinated aliphatic hydrocarbons. After completion of the phosgenation, the product, 1,6,11-undecanetriisocyanate may be isolated merely by distillation from the reaction medium. However, in case trace amounts of impurities which have hydrolysable chlorine compounds are contained in the product, the distillation can be carried out in the presence of anhydrous bases such as calcium oxide, potassium carbonate, sodium carbonate, etc. to allow recovery of the isocyanate practically free from the chlorine compounds.

The triisocyanate thus produced has been found to have several unusual properties, and is believed to be a novel compound, which belongs to a new type of aliphatic triisocyanates. The triisocyanate of the present invention possesses advantages over aliphatic isocyanates of the prior art. This isocyanate differs significantly from the other aliphatic isocyanates, such as hexamethylene diisocyanate or 2′-isocyanatoethyl-6-isocyanatocaproate because of its substantially reduced toxicity and strong resistance to the action of alkaline substances. Some of these properties may be attributed to the chemical structure of the novel isocyanate as it has neither any hetero-atoms such as oxygen and nitrogen, nor any unsaturated bonds in the molecule except for the three NCO groups. The novel isocyanate of the present invention boils at 166°C to 167°C under a vacuum of 0.2 torr. This range of boiling point will be appreciated to be well-balanced from the view-point of purification and toxicity: this isocyanate generates scarcely any odour or vapour irritating to the nose, eyes, throat, etc, because of its low vapour pressure, whereas it can be easily purified by a vacuum distillation.

Moreover, the NCO group content in the novel triisocyanate of the present invention reaches as high as about 45 weight percent, based on the total weight of the triisocyanate. Furthermore, the novel triisocyanate has low viscosity so that it does not necessarily require a process to dilute it with solvents for the purpose of lowering viscosity for practical usage. Before this invention, we have not known of any aliphatic polyisocyanates possessing such high purity, low toxicity, high NCO content and moderately low viscosity.

The novel isocyanate of the present invention may be used in the same fields as aliphatic

polyisocyanates of prior art have been used. For example, it can be used for polyurethanes by reacting it with various compounds including polymers, containing active hydrogen groups such as polyols, and as an intermediate for producing other novel compounds or polymers, and so on. Polyurethanes are used for paints, films, foams, elastomers, adhesives, treating agents for textiles, and so on. Especially, the employment of the novel triisocyanate for producing polyurethanes brings satisfactory advantages to the products with respect to durability, water resistance, resistance to acids or bases as well as organic solvents and light-stability in comparison with that derived from available aliphatic polyisocyanates.

Polyurethanes can be prepared by the reaction of the triisocyanate of this invention with various polyols. The polyols may be monomeric or polymeric compounds having two or more hydroxyl groups. Monomeric polyols may be diols, triols, tetrols, pentols, hexols and their mixtures. Examples of such polyols are ethyleneglycol, propyleneglycol, diethyleneglycol, dipropyleneglycol, 1,4-butyleneglycol, 1,3-butyleneglycol, polyethyleneglycol, polypropyleneglycol, polybutyleneglycol, glycerine, trimethylolpropane, pentaerythritol, sorbitol, 2-methylglucoside, 1,2,6-hexanetriol, etc.

Polymeric polyols may be polyester, polyether, polyester-polyether polyols and various polymers prepared from monomers having a hydroxyl group or a functional group by which hydroxyl groups can be introduced into the polymer. Polyetherpolyols may be prepared by polymerizing ethyleneoxide or propyleneoxide with polyols such as glycerine, propyleneglycol and polyfunctional compounds such as ethylenediamine, or ethanolamine. Polyesterpolyol may be prepared by polycondensation of carboxylic acids such as adipic acid, dimer acid, phthalic anhydride and isophthalic acid with a polyol such as ethyleneglycol, propyleneglycol and trimethylolpropane. Other various polymeric polyols may be prepared from a polymerizable monomer having at least one hydroxyl group optionally with other copolymerizable monomers. The monomer having at least one hydroxyl group may be, for example, 2-hydroxyethyl acrylate, 2-hydroxypropylacrylate, 2-hydroxybutyl acrylate, 2-hydroxyethyl methacrylate, 2-hydroxypropyl methacrylate, 2-hydroxypentyl methacrylate, glycerine monomethacrylate, trimethylolpropane monoacrylate, 2-hydroxy-3-chloropropyl acrylate and 2-hydroxy-3-chloropropyl methacrylate. Other monomers copolymerizable with above hydroxyl group containing monomers may be

(1) acrylic acid or an alkyl ester thereof such as methyl acrylate and 2-ethylhexyl acrylate,

(2) methacrylic acid or an alkyl ester thereof such as ethylmethacrylate, decyl methacrylate and lauryl methacrylate,

(3) styrene or a derivative thereof such as $\alpha$-methyl styrene, $\beta$-chlorostyrene,

(4) vinyl esters such as vinyl acetate, vinyl propionate and vinyl isopropionate,

(5) nitriles such as acrylonitrile, and methacrylonitrile.

Further examples of hydroxyl group containing monomers may be addition products of acrylic or methacrylic acid with a monoepoxy compound or epoxy resin which are, for example, commercially available from Shell Chemical Corp. under the trade marks "Cardula E" and "Epikote 1001".

Preferable hydroxyl group containing monomers may be 2-hydroxyethyl methacrylate, 2-hydroxyethyl acrylate and 2-hydroxypropyl methacrylate, and preferable comonomers may be methyl acrylate, ethyl acrylate, butyl acrylate, methyl methacrylate, butyl methacrylate, lauryl methacrylate, acrylic acid, methacrylic acid, styrene, acrylamide, vinyl acetate, etc.

Preferable polymeric polyols may be prepared by polymerizing a monomer mixture having a composition as described below.

| | % By weight |
|---|---|
| A. Hydroxyalkyl acrylate or methacrylate | 5 to 30 |
| B. Alkyl acrylate or methacrylate | 50 to 95 |
| C. Optional other monomers | 0 to 50 |
| D. Acrylic or methacrylic acid | 0 to 10 |

When the amount of hydroxyalkyl acrylate or methacrylate is less than 5% by weight, the degree of crosslinking in polyurethane is too small and the film prepared therefrom does not have excellent physical properties.

The above monomer composition can be polymerized by various conventional polymerization processes such as solution, bulk, emulsion and dispersion polymerizations. Usually solution polymerization may be adopted.

The molecular weight of the polymeric polyols can be varied according to the usage of polyurethane prepared therefrom. For a polyurethane paint of higher solid content, polymeric polyols having a molecular weight of 500 to 5,000, preferably 500 to 3,000, are used. More specifically the preferable molecular weight is 500 to 1,000, when the polymeric polyol is a polyester type polyol, while the preferable molecular weight is 1,000 to 3,000, when the polymeric polyol is a polyacrylate type polyol.

Polyols usable in the present invention are not restricted to the above described examples. Various types of polyols can be used, either alone or in combination with others, in the present invention, as long as hydroxyl groups of those polyols can be reacted with triisocyanate to form urethane linkages. The preferred hydroxyl value may be 20 to 1000, more preferable 20 to 500. For the

preparation of high-solid type compositions a hydroxyl value of 50 to 1,000 may be especially suitable. When the hydroxyl value of the polyol is less than 20, the degree of crosslinking is too small to give the polyurethane desirable physical properties such as resistance to solvent or water and weatherability. When the hydroxyl value is more than 1,000, the degree of crosslinking becomes too large, and thereby only brittle coating film is obtained from the polyurethane coating.

Polyurethanes of the present invention can be prepared by mixing the above defined novel triisocyanate and various polyols, optionally in an organic solvent. Properties of the polyurethane may be variable depending upon the kind of polyol and NCO/OH ratio in the mixture. When film is to be prepared from the polyurethane, the NCO/OH ratio may preferably be controlled within the range of 0.5 to 2.0. For polyurethane paint the NCO/OH ratio may preferably be 0.5 to 1.2. In the field of electric insulation, capsules or mouldings, a composition within the NCO/OH ratio of 0.5 to 1.0 is preferably used. A composition having a NCO/OH ratio of 0.1 to 0.7 may be suitable for high performance adhesives or hardeners, whilst a composition having a larger NCO/OH ratio may preferably be utilized for polyurethane foam.

The reaction temperature for forming polyurethanes according to the present invention may be 20 to 160°C. The desirable reaction is naturally the urethane bond-forming reaction between isocyanate and hydroxyl groups, but other reactions between isocyanate groups and active hydrogen donating groups other than hydroxyl groups are not necessarily excluded. Such active hydrogen donating groups may be amines, carboxylic acids, substituted ureas, urethanes and amides. The isocyanate group may form a allophanate bond by reaction at a relatively high temperature with a urethane bond which has been formed by reaction between isocyanate and hydroxyl groups. Similarly a biuret bond may be formed from urea and isocyanate, and an acylurea bond from amide and isocyanate. These reactions which may possibly take place in the composition of the present invention can be utilized according to the usage of the final polyurethane products.

The polyurethane composition of the present invention, which contains the novel triisocyanate defined above, is exceedingly suitable for polyurethane paint. Polyurethane paints have been known to provide excellent coating films which have a strong adhesion to various base surfaces, a good balance between hardness and flexibility, anticracking property, resistance to water and chemicals and an excellent surface gloss. The novel polyurethane coatings provided by the present invention have, in addition to the above excellent properties of the known polyurethane coatings, the following remarkable characteristics:

(1) Odourlessness in handling because of the low vapour pressure and the absence of a content of free volatile monomer.

(2) A finish with a higher gloss level can be obtained, particularly resulting from the coating having a lesser bad influence on the surfacer while spraying of the top coat, due to the use of a thinner with a greater amount of aromatic hydrocarbon.

(3) Improved acid resistance and water resistance of finish can be achieved.

(4) Higher solid contents during spraying operations can be obtained, resulting in the use of a lesser amount of thinner, due to the low viscosity of the novel triisocyanate of this invention. This leads to a favourable situation with respect to anti-pollution, saving raw material and saving energy.

The polyurethane coating compositions of the present invention can be used in the form either of a two component type or of a one component type in the manner of known polyurethane coating compositions.

Coating can be carried out by conventional methods such as spray coating, brush coating, dipping or roller coating. The coating compositions may contain a proper amount of various pigments, plasticisers and other conventional additives. A catalyst accelerating the drying or hardening of the coating composition can also be used.

The polyurethane resins of the present invention can be used for moulded resinous articles or as paints in the field of automobiles, railway rolling stock, containers, aeroplanes, seamless floors, coil coatings, ships, various wooden articles and plastics.

The following examples will further serve to illustrate the present invention.

Example 1

In a 100 ml autoclave were placed 14 g of 7-(5′-aminopentyl)-3,4,5,6-tetrahydro-2H-azepine, 30 ml of 28% aqueous ammonia and 3 ml of Raney nickel. After the inside of the autoclave was flushed with hydrogen, the autoclave was pressurized with hydrogen until a pressure of $80\times10^5$ Pa was reached, and it was then heated at 90°C for 8 hrs. After the catalyst was filtered off, the water and ammonia were distilled off and the resulting residue was analysed by gas-chromatography and found to contain 13 g of 1,6,11-undecanetriamine and 1.8 g of 2-(5′-aminopentyl)-perhydroazepine. The residue was distilled at 132°C at 2 torr to give the pure triamine.

Example 2

To a solution of 100 g of 1,6,11-undecanetriamine obtained in Example 1 in 100 ml of methanol, 136 ml of concentrated (35 wt percent) hydrochloric acid was added dropwise while cooling to maintain the reaction temperature at below 30°C. The reaction mixture was concentrated by means of

a rotary evaporator in vacuo on a hot-water bath to yield a thick oil. The oil, which on digestion with 500 ml of isopropyl alcohol was similarly concentrated, was evacuated at about 80°C under a vacuum below 5 torr for 10 hrs to give white solids of 1,6,11-undecanetriamine trihydrochloride. The solids thus obtained were crushed and pulverized in a mortar with a pestle under a dry atmosphere to give a fine powder of below 175 $\mu$m diameter by passing through a screen of 80 mesh.

A four-necked, round-bottomed flask fitted with a mechanical stirrer, a thermometer, a gas inlet almost reaching the bottom of the flask and a condenser was charged with 66.5 g of the triamine hydrochloride powder and 665 ml of o-dichlorobenzene. The mixture was phosgenated by using a phosgene flow of approximately 30 g/hr. The reaction was started at 130°C and gradually heated to 140°C after 4 hrs, then maintained for 7 hours at 140°C and further for 4 hrs at 150°C. As the reaction proceeded, the powders which at the first were suspended in the mixture, were dissolved. After cooling to room temperature and filtration, the solvent was distilled off at about 40°C under reduced pressure of about 4 torr and the product was distilled at 166°C to 167°C at 0.2 torr to give 47.2 g of 1,6,11-undecanetriisocyanate:

$n_D^{20}$ 1.4720

Analysis—Calculated for $C_{14}H_{21}N_3O_3$ (percent): C 60.19; H 7.58; N 15.05, Found C 59.89; H 7.55; N 14.82

High-resolution MS—Calculated for $C_{14}H_{21}N_3O_3$, $M^+/e$=279.1584, Found $M^+/e$=279.1585

IR Spectra ($CM^{-1}$)—2940, 2860, 2260 (NCO), 1460, 1360

NMR Spectra (ppm)—1.45 (singlet, 16H), 3.4 (distorted triplet, 5H).

Example 3

In a four-necked, round-bottomed 3-litre flask on an ice-water bath, charged with 1.5 l of o-dichlorobenzene and phosgene generated by decomposing 740 g of trichloromethyl chloroformate over activated carbon, was added a solution of 300 g of 1,6,11-undecanetriamine in 200 ml of o-dichlorobenzene during 2 hrs with mechanical stirring. Then the mixture was heated to 70°C on an oil bath and stirred while being treated with a phosgene flow at 70°C for 30 min. The stirring of the resulting mixture was continued at 130°C for 2 hours, at 140°C for 2 hrs, and finally at 150°C for 2.5 hrs, while phosgene was blown through the mixture. After cooling the mixture and filtering it the solvent was distilled off and the product was distilled at 189°C at 0.6 torr to give 236 g of the crude desired isocyanate. 77 g of the crude product was distilled in the presence of 0.8 g of potassium carbonate at 168°C at 0.2 torr to give the pure isocyanate. Alternatively, 25 g of the crude product was distilled in the presence of 0.25 g of calcium carbonate at 179—180°C at 0.5 torr to give 22 g of the pure isocyanate.

Example 4

As mentioned in Example 2, into a solution of 300 g of the triamine in 300 ml methanol was added 430 ml of 36 wt percent of concentrated hydrochloric acid drop by drop for 3 hrs, while cooling below 30°C with mechanical stirring. The mixture, mixed with 300 ml of iso-propyl alcohol, was subjected to distillation at 50—60°C under reduced pressure by means of a rotary evaporator. The product, mixed with 200 ml methanol and 300 ml iso-propyl alcohol, was subjected to distillation in the same manner and on repeating distillation once more the product was evacuated at 80°C at 2 torr for 8 hrs to give white solids. The solids were pulverized and sieved by being passed through a screen of 60 mesh.

A four-necked, round-bottomed 4-litre flask was charged with 2.2 l of o-dichlorobenzene and 350 g of the triamine trihydrochloride produced above. The mixture was phosgenated for 13.5 hrs by using a flow of phosgene generated by decomposing 1.5 l of trichloromethyl chloroformate over activated carbon, while the reaction temperature was maintained at 130°C for 3 hrs, then at 140°C for 3.5 hours and further at 150°C for 7 hrs. After distillation of the solvent in the same manner as described in Example 2, 255.6 g of the triisocyanate was distilled at 183—189°C at 0.6 torr.

Examples 5—11

Each component shown in Table 1 was mixed with the isocyanate compound. The resulting liquids were coated onto tin panels, and then the coated panels were cured at 120°C for 2 hrs. The resulting films had the basic property shown and in addition exhibited an excellent gloss.

TABLE 1

| Example | Isocyanate compound | Polyol component | NCO/OH (mole ratio) | Property |
|---|---|---|---|---|
| 5 | 1,6,11-Undecane triisocyanate | Trimethylol propane | 1.0 | Hard |
| 6 | 1,6,11-Undecane triisocyanate | Glycerol | 1.0 | Hard Tough |
| 7 | 1,6,11-Undecane triisocyanate | 1,2,6-Hexanetriol | 1.0 | Hard |
| 8 | 1,6,11-Undecane triisocyanate | Polypropyleneglycol (Mn=410) | 1.0 | Soft Elastic |
| 9 | 1,6,11-Undecane triisocyanate | Trimethylol propane | 1.0 | Hard |
| 10 | 1,6,11-Undecane triisocyanate | Glycerol | 1.0 | Hard Tough |
| 11 | 1,6,11-Undecane triisocyanate | Polypropyleneglycol (Mn=2,000) | 1.0 | Soft Elastic |

Example 12

A vessel equipped with a mechanical stirrer, a thermometer, a reflux condenser and a nitrogen gas inlet tube, was charged with the following solvents under an atmosphere of nitrogen, and heated to 90—95°C.

| | |
|---|---|
| Xylol | 50 parts |
| Butylacetate | 50 parts |

The following mixture was dripped in at a constant rate over a period of 3 hours.

| | |
|---|---|
| Styrene | 34.0 parts |
| n-Butylacrylate | 38.0 parts |
| β-Hydroxyl methacrylate | 23.4 parts |
| Acrylic acid | 0.4 parts |
| Azobisisobutyronitrile | 1.2 parts |

After completion of dripping of the mixture, the mixture was maintained at 90—95°C for one hour, and then 0.7 parts of azobisisobutyronitrile were added 4 times at intervals of 30 minutes, and after that the mixture was held for further one hour.

This resulting solution of acrylic polyol was clear liquid, its Gardner Viscosity was T—U at 25°C, and the total solids content was 50%. The OH value of this solution was 50 and the calculated average OH value per one copolymer molecule was 12.9. Molecular weight (Mn) of this copolymer was 14,500.

This solution of the acrylic polyol was mixed with $TiO_2$ (titanium oxide) "R-930" (Trademark of Ishihara Sangyo Co. Ltd.) at 50% by weight of pigment weight content (PWC) using a paint conditioner (shaker).

The polyol component thus obtained was mixed with 1,6,11-undecanetriisocyanate at an NCO/OH ratio of 1.0, and thereby white enamel was prepared. This white enamel was diluted with a thinner composed of toluol and cellosolve-acetate (5)/50 wt%) to a settlement time of 18 seconds using Ford Cup No. 4. This diluted composition was sprayed onto a zinc phosphate treated steel coated with the primer surfacer No. 114 (Kansai Paint Co. Ltd.) and polished with sand paper, to form a smooth film at a dry film thickness of about 40 μm. Panels thus coated were cured at room (23°C) for 7 days The resulting film was tough and had excellent properties of acid resistance and warm water resistance as shown in Table 2. Also the exposure test results indicated no significant difference in yellowing resistance between the said film and the coating film obtained by using the available aliphatic polyisocyanate, especially that sold under the trade mark Desmodur N-75 (Bayer AG).

The same procedure as mentioned above was repeated without using $TiO_2$. The evaluation data of the diluted composition and the obtained clear film were summarized in Table 3.

Example 13.

The apparatus of Example 12 was charged with the following solvents under an atmosphere of nitrogen, and heated to 80—85°C.

| | |
|---|---|
| Xylol | 80 parts |
| Butylacetate | 20 parts |

The following mixture was dripped in at a constant rate over a period of 3 hours.

| | |
|---|---|
| Styrene | 25.0 parts |
| Methyl methacrylate | 25.0 parts |
| n-Butylmethacrylate | 21.0 parts |
| n-Butylacrylate | 14.0 parts |
| β-Hydroxylmethacrylate | 12.0 parts |
| Acrylic acid | 0.7 parts |
| Azobisisobutyronitrile | 1.2 parts |

On completion of dripping of the mixture, the mixture was maintained at 80—85°C for 2 hours, and then 0.5 parts of azobisisobutyronitrile were added 4 times at intervals of 2 hours, and after that the mixture was held for further 3 hours. This resulting solution of acrylic polyol was clear liquid and its Gardner Viscosity was V—W at 25°C; the total solids content was 50%. The molecular weight (Mn) of the copolymer was 11,700, and the OH value of the solution was 25, the accordingly calculated average OH value per one copolymer molecule being 5.2.

Using the same method as described in Example 12, a white enamel was prepared by mixing the polyol solution obtained above with $TiO_2$ R-930 and 1,6,11-undecanetriisocyanate. This enamel was coated, and then cured. The resulting film exhibited excellent qualities such as uniform high gloss, acid resistance and warm water resistance as shown in Table 2. The yellowing resistance of the exposure test film was good, equivalent to that of the film obtained by using the available aliphatic polyisocyanate.

The same procedure was repeated without using $TiO_2$, and there was thus prepared a clear enamel and a clear coating film. The evaluation data thereof are summarized in Table 3.

Comparative Examples 1 and 2

In these examples four enamels, two white and two clear, were made in the same way as Examples 12 and 13, but using "Desmodur N-75" at an NCO/OH ratio of 1.0 in place of the 1,6,11-undecanetriisocyanate used in Examples 12 and 13.

The white enamels thus obtained were diluted to a settlement time of 18 seconds using Ford Cup No. 4, and the diluted compositions were coated onto steel panels in the same way as was used for Example 12.

The properties of the white films are compared with those of Examples 12 and 13 in Table 2, and Table 3 shows a comparison between the clear composition and film of Examples 12 and 13 and those of the comparative examples made with "Desmodur N-75".

From the results summarized in Tables 2 and 3 the effect of the present invention can be understood as follows. The paints made using the triisocyanate of the present invention were less volatile, thus saving raw materials when spraying, and exhibited a good compatibility. The cured films had the same level of practical properties as those obtained with the use of "Desmodur N-75" and in particular exhibited a far better acid resistance.

TABLE 2

| Test | | Example 12 | Comparative Example 1 | Example 13 | Comparative Example 2 |
|---|---|---|---|---|---|
| Items | Condition | 1,6,11-undecanetriisocyanate | "Desmodur N-75" | 1,6,11-undecanetriisocyanate | "Desmodur N-75" |
| Initial hardness rate | | O | O | O | O |
| Gloss | 60° | 95 | 92 | 93 | 93 |
| Pencil hardness | | F | F | F | F |
| Impact resistance 1/2" (12.7 mm), 1000 g | Du Pont | 30 cm | 30 cm | 25 cm | 20 cm |
| Erichsen | | 7.0 mm | 6.5 mm | 7.7 mm | 7.5 mm |
| Adhesion | Cross Cut | 100/100 | 100/100 | 100/100 | 100/100 |
| Warm water resistance | 50°C×24 hr | O | Δ | O | Δ |
| Acid resistance | 40 vol% $H_2SO_4$ 55°C×5 hr | O | x | O | x |
| Solvent resistance | Naphtha No. 5 /toluol=6/4 dipping for 10 min | O | O | O | O |
| " | Xylol rubbing 30 times | O | O | Δ | Δ |
| Yellowing index (YI) | UV* 0 hr | 2.5 | 3.2 | 2.8 | 3.0 |
| " | " 200 hr | 10.0 | 12.1 | 9.6 | 11.2 |
| YI | 200 hr | 7.6 | 8.9 | 7.4 | 8.2 |
| Δ E (Lab) | 200 hr | 5.0 | 5.5 | 5.5 | 5.0 |

*Sterilization Lamp GL 15 (15 W) made by Tokyo Shibaura Electric Co., Ltd.,
Wave length: 254 mm,
radiation strength from distance 20 cm: 600 $\mu W/cm^2$
Pigmentation: PWC 50%
Curing condition: 23°C×7 days
Evaluated level:
Sign ◎ excellent
O good
Δ fair
x poor.

In the above table ΔE is a measure of colour difference based on the Hunter equation (Lab).

TABLE 3

| | Example 12 | Comparative Example 1 | Example 13 | Comparative Example 2 |
|---|---|---|---|---|
| Polyol OH value | 50 | 50 | 25 | 25 |
| Isocyanate | 1,6,11-Undecanetri-isocyanate | "Desmodur N-75" | 1,6,11-Undecanetri-isocyanate | "Desmodur N-75" |
| Compatibility toluol/cellosolve acetate 50:50 | O | O | O | O |
| Toluol | O | Δ (slightly opaque) | O | Δ (slightly opaque) |
| Film appearance* | ◎ | O | ◎ | O |
| Spraying solids content Ford Cup No. 4 20 seconds | 55% | 49% | 54% | 48% |

*Used a thinner comprising toluol and cellosolve acetate (50/50)
Pigmentation: PWC 50%
Curing condition: room temp (23°C)x7 days
Substrate: Zinc phosphate treated steel coated with primer surfacer No. 114 and polished with sand paper
Evaluated level sign: same as Table 2.

Example 14

Polyester polyol was prepared as follows:

| | |
|---|---|
| Neopentyl glycol | 126.9 parts |
| Trimethylol propane | 22.1 parts |
| Adipic acid | 72.3 parts |
| Isophthalic acid | 123.2 parts |

This mixture was charged into the vessel and heated to 200°C for 30 minutes with stirring. Then the mixture was maintained for about 15 minutes until the Gardner viscosity rose to F, while the acid value decreased to about 10 and the OH value decreased to about 100 as measured in the solution diluted with methyl ethyl ketone solvent to a non-volatile level of 60%. After that this mixture was cooled and diluted with methyl ethyl ketone solvent to a non-volatile level of 60% and thus a polyester polyol solution was obtained.

This solution mentioned above and 1,6,11-undecanetriisocyanate were uniformly mixed at a NCO/OH ratio of 1.0. This composition was coated and cured. The resulting film had a high gloss and excellent mechanical properties.

## Claims

1. 1,6,11-Undecane triisocyanate represented by following formula.

$$OCN{-}(CH_2)_5{-}\underset{\displaystyle NCO}{\underset{|}{CH}}{-}(CH_2)_5{-}NCO \qquad (I)$$

2. A method for producing 1,6,11-undecane triisocyanate (I) which comprises reacting 1,6,11-undecane triamine or a salt thereof with phosgene in an inert organic medium at a temperature of 60°C to 230°C.

3. A method for producing 1,6,11-undecane triisocyanate which comprises reacting 1,6,11-undecane triamine or a salt thereof with phosgene in an inert organic medium at a temperature below 40°C and pyrolyzing the resulting tris(carbamoyl chloride) in the presence of phosgene at a temperature of about 60°C to 230°C.

4. A method according to Claim 2 or Claim 3 wherein there is used the hydrochloric acid salt of 1,6,11-undecane triamine.

5. A method according to any one of Claims 2 to 4, wherein the 1,6,11-undecane triamine is prepared by catalytic hydrogenation of 7-(5'-aminopentyl)-3,4,5,6-tetrahydro-2H-azepine or bis(5-aminopentyl)ketone (or a salt thereof) in an aqueous solution of ammonia.

6. A method according to Claim 5, wherein there is used the hydrochloric acid salt of bis(5-aminopentyl)ketone.

7. A polyurethane prepared by the reaction of a polyol and 1,6,11-undecanetriisocyanate.

8. A polyurethane coating composition comprising mainly a polyol and 1,6,11-undecanetriisocyanate.

**Patentansprüche**

1. 1,6,11-Undecan-triisocyanat der folgenden Formel

$$OCN—(CH_2)_5—\underset{\displaystyle NCO}{\underset{|}{CH}}—(CH_2)_5—NCO \qquad (I)$$

2. Verfahren zur Herstellung von 1,6,11-Undecan-triisocyanat (I), bei dem 1,6,11-Undecan-triamin oder ein Salz hiervon mit Phosgen in einem inerten organischen Medium bei einer Temperatur von 60 bis 230°C umgesetzt wird.

3. Verfahren zur Herstellung von 1,6,11-Undecan-triisocyanat, bei dem 1,6,11-Undecan-triamin oder ein Salz hiervon mit Phosgen in einem inerten organischen Medium bei einer Temperatur unter 40°C umgesetzt und das anfallende Tris(carbamoylchlorid) in Gegenwart von Phosgen bei einer Temperatur von etwa 60°C bis 230°C pyrolysiert wird.

4. Verfahren nach Anspruch 2 oder Anspruch 3, bei dem das Salzsäuresalz von 1,6,11-Undecan-triamin verwendet wird.

5. Verfahren nach irgend einem der Ansprüche 2 bis 4, bei dem das 1,6,11-Undecan-triamin durch katalytische Hydrierung von 7-(5'-Aminopentyl)-3,4,5,6-tetrahydro-2H-azepin oder Bis(5-aminopentyl)keton (oder einem Salz hiervon) in einer wässrigen Ammoniak-Lösung hergestellt wird.

6. Verfahren nach Anspruch 5, bei dem das Salzsäuresalz von Bis(5-aminopentyl)keton verwendet wird.

7. Polyurethan, hergestellt durch Umsetzen eines Polyols und von 1,6,11-Undecan-triisocyanat.

8. Polyurethan-Überzugsmittel, hauptsächlich ein Polyol und 1,6,11-Undecan-triisocyanat umfassend.

**Revendications**

1. 1,6,11-Undecane triisocyanate représenté par la formule qui suit:

$$OCN—(CH_2)_5—\underset{\displaystyle NCO}{\underset{|}{CH}}—(CH_2)_5—NCO \qquad (I)$$

2. Procédè de production de 1,6,11-undecane triisocyanate (I) qui consiste à faire réagir une 1,6,11-undecane triamine ou son sel avec du phosgène dans un milieu organique inerte à une température de 60 à 230°C.

3. Procédé de production de 1,6,11-undecane triisocyanate qui consiste à faire réagir une 1,6,11-undecane triamine ou son sel avec du phosgène dans un milieu organique inerte à une température inférieure à 40°C et à pyrolyser le tris-chloro de carbamoyl) résultant en présence d'un phosgène à une température de l'ordre de 60 à 230°C.

4. Procédé selon la revendication 2 ou la revendication 3 où l'on utilise le sel d'acide chlorhydrique de 1,6,11-undecane triamine.

5. Procédé selon l'une des revendications 2 à 4 où la 1,6,11-undecane triamine est préparée par hydrogénation catalytique de 7-(5'-aminopentyl)-3,4,5,6-tétrahydro-2H-asepine ou bis(5-aminopentyl) cétone (ou son sel) dans une solution aqueuse d'ammoniac.

6. Procédé selon la revendication 5 où l'on utilise le sel d'acide chlorhydrique de bis(5-aminopentyl) cétone.

7. Polyuréthane préparé par la réaction d'un polyol et de 1,6,11-undecanetriisocyanate.

8. Composition de revêtement de polyuréthane comprenant principalement un polyol et du 1,6,11-undecanetriisocyanate.